# EUROPEAN PATENT SPECIFICATION

(11) **EP 1 297 850 B1**
(45) Date of publication and mention of the grant of the patent: **19.08.2015**
(21) Application number: 01947821.3
(22) Date of filing: 04.07.2001
(51) Int. Cl.: A61K 45/06, A61K 38/09, A61K 38/08, A61K 31/519, A61P 5/24, C07D 495/04

(54) **MEDICINAL PREPARATIONS FOR TREATING SEX HORMONE-DEPENDENT DISEASES**
MEDIZINISCHE ZUSAMMENSETZUNGEN ZUR BEHANDLUNG VON MIT SEXUALHORMONEN VERBUNDENEN ERKRANKUNGEN
PREPARATIONS MEDICINALES POUR LE TRAITEMENT DES MALADIES DEPENDANT DES HORMONES SEXUELLES

(30) Priority: 05.07.2000 JP 2000208253
(43) Date of publication of application: 02.04.2003
(73) Proprietor: Takeda Pharmaceutical Company Limited, Osaka-shi Osaka 541-0045 (JP)
(72) Inventor: IGARI, Yasutaka, Kobe-shi Hyogo 658-0015 (JP); KAMEI, Shigeru, Takaraduka-shi Hyogo 665-0847 (JP)
(74) Representative: Jones, Nicholas Andrew
(86) International application number: PCT/JP2001/005808
(87) International publication number: WO 2002/002144

(56) References cited:
- WO-A1-97/22357
- WO-A1-97/40846
- AKAKURA KOICHIRO ET AL.: 'Effects of intermittent androgen suppression on androgen-dependent tumors' CANCER vol. 71, no. 9, 1993, pages 2782 - 2790, XP002942968
- HSUEH AARON J.W. ET AL.: 'Mechanism of the direct action of gonadotropin-releasing hormone and its antagonist on androgen biosynthesis by cultured rat testicular cells' ENDOCRINOLOGY vol. 112, no. 5, 1983, pages 1653 - 1661, XP002942969

## Description

### TECHNICAL FIELD

The present invention relates to (1) the use of a compound having a luteinizing hormone-releasing hormone agonistic effect or an acetate thereof and a compound having a luteinizing hormone-releasing hormone antagonistic effect or a pharmacologically acceptable salt thereof for the preparation of a medicinal preparation for treating sex hormone-dependent diseases.

### BACKGROUND ART

A luteinizing hormone-releasing hormone which is known as LHRH (or GnRH) is released from hypothalamus and binds to a receptor of glandula pituitaria. LH (luteinizing hormone) and FSH (follicle stimulating hormone) which are released due to this act on gonadal gland to synthesize a steroid sex hormone. The successive administration of a compound having a potent luteinizing hormone-releasing hormone agonistic effect results in the reduction of the number of the available receptor and the formation of a steroid sex hormone derived from gonadal gland is suppressed. By utilizing this, a compound having a luteinizing hormone-releasing hormone agonistic effect is clinically applied as medicaments for treatment of sex hormone-dependent diseases such as prostate cancer, benign prostatic hyperplasia, endometriosis, hysteromyoma, metrofibroma, precocious puberty, breast cancer.

As a treatment method comprising preventing and delaying the change of sex hormone-dependent diseases (especially prostate cancer) into sex hormone-independent, an intermittent treatment comprising administration cessation of a compound having a luteinizing hormone-releasing hormone agonistic effect for a drug starving, and exposing to a sex hormone with a sufficient concentration so as to maintain the disease being hormone-dependent in a treatable state, is used (for example, Cancer, 71(1993) 2782-2790).

As a compound having a luteinizing hormone-releasing hormone agonistic effect is an agonist for LHRH receptor, the concentrations of testosterone and estrogen are increased immediately after the first administration of a preparation of the compound, due to the glandula pituitaria gonad-stimulating effect which is inherent in the compound, and a temporary deterioration of a disease (flare phenomenon) is observed. After that, the number of the LHRH receptor is decreased and the treatment of the hormone dependent diseases becomes to be effective, while the number of LHRH receptor is not recovered quickly as long as the concentration of the compound in blood is more than a certain concentration, and therefore an excessive period is required in order to be exposed to a hormone having the concentration required for an intermittent treatment. Then, it has been desired to develop a treatment method which can definitely determine the drug cessation period in an intermittent treatment; or a treatment method in which the suppression of a sex hormone is achieved during the drug administration period, while more definite recovery of the sex hormone is accelerated after finishing the administration period.

WO9740846 relates to a pharmaceutical composition comprising a luteinizing hormone-releasing hormone agonist in combination with a luteinizing hormone-releasing hormone antagonist.

WO97/22357 relates to methods for treating prostate cancer. The methods disclosed generally feature administration to a subject of an LHRH antagonist, in combination with a second therapy.

### DISCLOSURE OF THE INVENTION

The present inventors have studied intensively to solve the above-mentioned problem. As a result, the present invention has been completed.

That is, the present invention relates to:
(1) Use of leuprorelin or an acetate thereof and a compound having a luteinizing hormone-releasing hormone antagonistic effect selected from NAcD2Nal-D4ClPhe-D3Pal-Ser-NMeTyr-DLys(Nic)-Leu-Lys(Nisp)-Pro-AlaNH2, N(4H2-furoyl)Gly-D2Nal-D4ClPhe-D3Pal-Ser-NMeTyr-DLys(Nic)-Leu-Lys(Nisp)-Pro-DAlaNH2, cetrorelix, ganirelix, antarelix, decirelix, azaline, antide, ramorelix and abarelix, or a pharmacologically acceptable salt thereof, for the preparation of a medicinal preparation for treating sex hormone-dependent diseases selected from prostate cancer, uterine cancer, breast cancer, glandula pituitaria tumor, benign prostatic hyperplasia, endometriosis, hysteromyoma, precocious puberty,, dysmenorrhea, amenorrhea, premenstrual syndrome and multilocular ovarian syndrome, wherein leuprorelin or an acetate thereof is to be administered followed by the compound having a luteinizing hormone-releasing hormone antagonistic effect at the interval of not less than 1 day;
(2) The use according to the above (1), wherein the compound having a luteinizing hormone-releasing hormone antagonistic effect is NAcD2Nal-D4ClPhe-D3Pal-Ser-NMeTyr-DLys(Nic)-Leu-Lys(Nisp)-Pro-DAlaNH₂ or a pharmacologically acceptable salt thereof;
(3) The use according to the above (1), wherein the leuprorelin or an acetate thereof is used as an injectable preparation;
(4) The use according to the above (1), wherein the leuprorelin or an acetate thereof is used as a sustained release preparation;
(5) The use according to the above (1), wherein the leuprorelin or an acetate thereof is used as a preparation for nasal administration;
(6) The use according to the above (1), wherein the compound having a luteinizing hormone-releasing hormone antagonistic effect is used as an injectable preparation;
(7) The use according to the above (1), wherein the compound having a luteinizing hormone-releasing hormone antagonistic effect is used as a sustained release preparation;
(8) The use according to the above (1), wherein the compound having a luteinizing hormone-releasing hormone antagonistic effect is used as a preparation for nasal administration;
(9) The use according to the above (1), wherein the medicinal preparation is for maintaining a hormone therapy;
(10) The use according to the above (1), wherein the medicinal preparation is for determining a drug cessation period;
(11) The use according to the above (1), wherein the medicinal preparation is for recovering the concentration of a sex hormone in a living body;
(12) The use according to the above (1), wherein the medicinal preparation is for maintaining sex hormone-dependency;
(13) The use according to the above (1), wherein the sex hormone-dependent disease is prostate cancer.

### BEST MODE FOR CARRYING OUT THE INVENTION

In the present description, the sex hormone-dependent diseases mean diseases selected from sex hormone-dependent cancer (e.g., prostate cancer, uterine cancer, breast cancer, glandula pituitaria tumor), benign prostatic hyperplasia, endometriosis, hysteromyoma, precocious puberty, dysmenorrhea, amenorrhea, premenstrual syndrome, niultilocular ovarian syndrome.

The compound having luteinizing hormone-releasing hormone antagonistic effect used in the present invention is a peptide compound.

In the present invention, the peptide compound having a luteinizing hormone-releasing hormone agonistic effect is represented by 5-oxo-Pro-His-Trp-Ser-Tyr-DLeu-Leu-Arg-Pro-NH-C₂H₅ (leuprorelin) or an acetate thereof.

In the present invention, the peptide represented by NAcD2Nal-D4ClPhe-D3Pal-Ser-NMeTyr-DLys(Nic)-Leu-Lys(Nisp)-Pro-DAlaNH₂) is preferred as the compound having a luteinizing hormone-releasing hormone antagonistic effect.

Furthermore, specific examples of the peptide compound having a luteinizing hormone-releasing hormone antagonistic effect include, NAcD2Nal-D4ClPhe-D3Pal-Ser-NMeTyr-DLys(Nic)-Leu-Lys(Nisp)-Pro-DAlaNH₂, N(4H2-furoyl)Gly-D2Nal-D4ClPhe-D3Pal-Ser-NMeTyr-DLys(Nic)-Leu-Lys(Nisp)-Pro-DAlaNH₂, cetrorelix, ganirelix, antarelix, decirelix, azaline, antide, ramorelix, abarelix, and pharmaceutically acceptable salts thereof.

These peptides can be prepared by methods as described in Treatment with GnRH analogs: Controversies and perspectives (The Parthenon Publishing Group Ltd., published in 1996), JP 3-503165 A, JP 3-101695 A, JP 7-97334 A and JP 8-259460 A or similar methods.

The compound having a luteinizing hormone-releasing hormone antagonistic effect used in the present invention may be the free compound itself or a pharmacologically acceptable salt thereof.

Examples of the salt include, when the compound having a luteinizing hormone-releasing hormone antagonistic effect has a basic group such as an amino group, a salt with an inorganic acid (also referred to an inorganic free acid) (e.g., carbonic acid, bicarbonic acid, hydrochloric acid, sulfuric acid, nitric acid, boric acid), an organic acid (also referred to an organic free acid) (e.g., succinic acid, acetic acid, propionic acid, trifluoroacetic acid).

When the biologically active substance has an acidic group such as a carboxyl group, examples of the salt include a salt with an inorganic base (also referred to an inorganic free base) (e.g., an alkaline metal such as sodium, potassium, an alkaline earth metal such as calcium, magnesium) or an organic base (also referred to an organic free base) (e.g., an organic amine such as triethylamine , a basic amino acid such as arginine). Further, the physiologically active peptide may form a metal complex compound (e.g., copper complex, zinc complex).

Specifically, both of the above-mentioned peptide represented by NAcD2Nal-D4ClPhe-D3Pal-Ser-NMeTyr-DLys(Nic)-Leu-Lys(Nisp)-Pro-DAlaNH₂ and the peptide represented by 5-oxo-Pro-His-Trp-Ser-Tyr-DLeu-Leu-Arg-Pro-NH-C₂H₅ (leuprorelin) are preferably used as acetates.

The compound having a luteinizing hormone-releasing hormone agonistic effect or an acetate thereof or the compound having a luteinizing hormone-releasing hormone antagonistic effect or a pharmacologically acceptable salt thereof may be used for oral administration as a tablet optionally having sugar coating, a capsule, an elixir, a sustained release preparation, or an aseptic solution in water or a pharmaceutically acceptable liquid other than water, or may be used for parenteral administration as an injectable preparation such as a suspension, a sustained release preparation, or as a preparation for nasal administration such as a solution, a suspension. The above-mentioned preparations may be prepared, for example, by mixing the compound having a luteinizing hormone-releasing hormone agonistic effect or an acetate thereof or the compound having a luteinizing hormone-releasing hormone antagonistic effect or a pharmacologically acceptable salt thereof with a physiologically acceptable known carrier, flavor, excipient, vehicle, antiseptic agent, stabilizer, binder, in a unit dosage form according to generally required and accepted pharmaceutical practice.

The additives which may be added to a tablet, a capsule include, for example, a binder such as gelatin, corn starch, tragacanth, gum arabic, an excipient such as crystalline cellulose, a swelling agent such as corn starch, gelatin, arginic acid, a lubricant such as magnesium stearate, a sweetening agent such as sucrose, lactose or saccharin, a flavor such as pepper mint, Akamono oil or cherry. When the preparation in the form of unit dosage is a capsule, a liquid carrier such as a fat and oil may be further added to the materials of the above-mentioned type. An aseptic injectable composition may be formulated according to a conventional pharmaceutical practice such as dissolution or suspension of an activating substance in a vehicle such as water for injection, a naturally occurring vegetable oil such as sesame oil, palm oil. Examples of an aqueous injectable solution include an isotonic solution containing saline, glucose or another adjuvant (e.g., D-sorbitol, D-mannitol, sodium chloride, etc.), and may be used in combination with a suitable dissolution aid, for example, alcohol (e.g., ethanol), polyalcohol (e.g., propylene glycol, polyethylene glycol), nonionic surfactant (e.g., Polysolbate 80 (TM), HCO-50). Examples of an oily liquid include sesame oil, soybean oil, and may be used in combination with benzyl benzoate, benzyl alcohol, which are dissolution aids. The preparation may also be admixed with, for example, a buffer (for example, phosphorate salt buffer solution, sodium acetate buffer solution), a soothing agent (for example, benzalconium chloride, procaine hydrochloride), a stabilizer (for example, human serum albumin, polyethylene glycol), a preservative (for example, benzyl alcohol, phenol), an antioxidant. The thus-prepared injectable solution is normally filled in a suitable vial or ampoule.

Especially, the compound having a luteinizing hormone-releasing hormone agonistic effect or an acetate thereof or the compound having a luteinizing hormone-releasing hormone antagonistic effect or a pharmacologically acceptable salt thereof is preferably used as a preparation for nasal administration, or an injectable preparation such as a sustained release preparation.

The sustained release preparation (preferably sustained release injectable preparation) containing the compound having a luteinizing hormone-releasing hormone agonistic effect or an acetate thereof or the compound having a luteinizing hormone-releasing hormone antagonistic effect or a pharmacologically acceptable salt thereof may be prepared according to a method known per se, for example, the method described in JP 60-100516 A, JP. 62-201816 A, JP 4-321622 A, JP 6-192068 A, JP 9-132524 A, JP 9-221417 A, JP 11-279054 A, WO99/360099.

The oral preparation, injectable and nasal preparation of the compound having a luteinizing hormone-releasing hormone agonistic effect or an acetate thereof or the compound having a luteinizing hormone-releasing hormone antagonistic effect or a pharmacologically acceptable salt thereof may also be prepared according to a known method or a similar method thereto.

In the present invention, the compound having a luteinizing hormone-releasing hormone agonistic effect or an acetate thereof or a preparation containing the compound (preferably a sustained release preparation) is administered, followed by administering the compound having a luteinizing hormone-releasing hormone antagonistic effect or a pharmacologically acceptable salt thereof or a preparation containing the compound (preferably a sustained release preparation), within the medicable period of the compound having a luteinizing hormone-releasing hormone agonistic effect or an acetate thereof, wherein the administration interval of each compound or salts thereof or a preparation containing the compound is not less than 1 day and may be suitably selected from the medicable periods of the compound having a luteinizing hormone-releasing hormone agonistic effect. For example, when a sustained release preparation containing the compound having, a luteinizing hormone-releasing hormone agonistic effect or an acetate thereof is used, the medicable period of the compound having a luteinizing hormone-releasing hormone agonistic effect or an acetate thereof is generally not less than 7 days and up to 12 months, preferably not less than 10 days and up to 6 months, more preferably not less than 14 days and up to 4 months, and may be suitably selected from these medicable periods.

During the required treatment period, the compound having a luteinizing hormone-releasing hormone agonistic effect or an acetate thereof or a preparation containing the compound (preferably a sustained release preparation) to be administered in advance may be administered successively at administration intervals as determined by the medicable period thereof. While the compound having a luteinizing hormone-releasing hormone antagonistic effect or a pharmacologically acceptable salt thereof or a preparation containing the compound (preferably a sustained release preparation) is used as the final administration in a series of treatment, it may be administered successively in order to ensure the determination of the drug cessation period.

In case of the sustained release preparation, the medicable period may be determined by measurement of a sex hormone (e.g., testosterone, LH, FSH, estrogen) concentration in a living body or the concentration of PSA by a method known per se such as EIA, RIA or a similar method thereto.

The compound having a luteinizing hormone-releasing hormone agonistic or antagonistic effect or a pharmacologically acceptable salt thereof may be administered to a mammal (e.g., human, monkey, dog, cow, sheep, swine, mouse, rat) as a safe and low-toxic medicament.

While a dosage of the compound having a luteinizing hormone-releasing hormone agonistic or antagonistic effect or a pharmacologically acceptable salt thereof varies depending on an objective disease, an objective animal, a dosage of a single dose may be selected from, for example, preferably the range of about 0.01 mg to 100 mg/kg body weight per an adult patient of prostate cancer. More preferably, it may be selected from the range of about 0.05 mg to 50 mg/kg body weight.

While a dosage of a sustained release preparation containing the compound having a luteinizing hormone-releasing hormone agonistic or antagonistic effect or a pharmacologically acceptable salt thereof varies depending on particular kind and content, particular dosage form of the compound having a luteinizing hormone-releasing hormone agonistic or antagonistic effect or a pharmacologically acceptable salt thereof as the basis, the release lasting time of the basis, the objective, disease, the objective animal, for example, in case of a sustained release preparation having a medicable period of 1 month, a dosage in a single dose of the sustained release preparation may be suitably selected from the range of, preferably about 0.1 mg to 500 mg/kg body weight per an adult patient of prostate cancer. More preferably, it may be suitably selected from the range of about 0.2 mg to 300 mg/kg body weight.

As shown in the following Example 1, when the compound having a luteinizing hormone-releasing hormone antagonistic effect or a pharmacologically acceptable salt thereof or a preparation containing the compound (preferably a sustained release preparation) is administered after administration of the compound having a luteinizing hormone-releasing hormone agonistic effect or an acetate thereof or a preparation containing the compound (preferably a sustained release preparation), the concentration of sex hormones (e.g., testosterone, LH, FSH, estrogen) is recovered quickly after the medicable period of the compound having a luteinizing hormone-releasing hormone antagonistic effect or a pharmaceutically acceptable salt thereof. Therefore, the use method of the present invention therefore makes it possible to definitely determine the drug cessation period in an intermittent treatment.

That is, a method for determining a drug cessation period, and a method for recovering the concentration of a sex hormone (e.g., testosterone, LH, FSH, estrogen) in a living body, wherein a preparation containing a compound having a luteinizing hormone-releasing hormone antagonistic effect or a pharmaceutically acceptable salt thereof (preferably a sustained release preparation) is used (preferably, a combination of a preparation containing a compound having a luteinizing hormone-releasing hormone agonistic effect or an acetate thereof (preferably a sustained release preparation) and a preparation containing a compound having a luteinizing hormone-releasing hormone antagonistic effect or a pharmaceutically acceptable salt thereof (preferably a sustained release preparation) is used) is described.

In the present disclosure, the drug cessation period can be determined by measuring the concentration of a sex hormone (e.g., testosterone, LH, FSH, estrogen) in a living body or the concentration of PSA. The drug cessation period means a period from the time when the concentration of a sex hormone in a living body is recovered to a similar concentration before administration of a preparation containing a compound having a luteinizing hormone-releasing hormone agonistic effect or an acetate thereof (normal state) to the time of the next administration.

The recovery of the concentration of a sex hormone in a living body in the present invention may be determined by a criteria for measurement of a sex hormone in normal medically practice. As the concentration of a sex hormone in a living body varies depending on a particular measurement method, time for collection of blood, it is difficult to define "recover" unambiguously and numerically. However, for example, not less than about 50%, preferably not less than about 60%, more preferably not less than about 70% of the concentration before administration of a preparation containing a compound having a luteinizing hormone-releasing hormone agonistic effect or an acetate thereof (normal state) is referred to be "recovered".

Thus, by using a preparation containing a compound having a luteinizing hormone-releasing hormone antagonistic effect or a salt thereof, the concentration of a sex hormone in a living body which has been reduced by administration of a preparation containing a compound having a luteinizing hormone-releasing hormone agonistic effect or an acetate thereof may be restored quickly, which makes it possible to more definitely and shortly determine the drug cessation period.

Furthermore, by using a compound having a luteinizing hormone-releasing hormone antagonistic effect or a salt thereof or a preparation containing the compound (preferably a sustained release preparation), the concentration of a sex hormone (e.g., testosterone, LH, FSH, estrogen) in a living body is recovered quickly, which makes it possible to maintain the sex hormone-dependent disease being hormone-dependent due to the drug cessation period which is definitely produced. Thus, an agent for maintaining a hormone therapy containing a compound having a luteinizing hormone-releasing hormone antagonistic effect or a salt thereof is also described.

After the concentration of a sex hormone is recovered, a compound having a luteinizing hormone-releasing hormone agonistic effect or an acetate thereof or a preparation containing the compound and/or a compound having a luteinizing hormone-releasing hormone antagonistic effect or a salt thereof or a preparation containing the compound may be administered.

The concentration of a sex hormone (e.g., testosterone, LH, FSH, estrogen) during the medicable period or after the medicable period may be determined according to a method known per se (EIA, RIA) or a similar method thereto.

The meaning of the abbreviations as used in the present description is as follows.

| Abbreviation | Name |
|---|---|
| N(4H2-furoyl)Gly: | N-tetrahydrofroylglycine residue |
| NAc : | N-acetyl group |
| D2Nal : | D-3-(2-naphthyl)alanine residue |
| D4ClPhe : | D-3-(4-chloro)phenylalanine residue |
| D3Pal : | D-3-(3-pyridyl)alanine residue |
| NMeTyr : | N-methyltyrosine residue |
| DLys(Nic) : | D-(ε-N-nicotinoyl)lysine residue |
| Lys(Nisp) : | (ε-N-isopropyl)lysine residue |

When the other amino acids are represented by abbreviations, these are based on the abbreviations by IUPAC-IUB Commission of Biochemical Nomenclature (European Journal of Biochemistry, Vol.138. pp.9-37, 1984) or conventional abbreviations in the art. With regard to amino acids, when an optical isomer is exist, it represents L form unless otherwise mentioned.

### EXAMPLES

The present invention is illustrated in more detail with reference to the following Reference Example and Example, but these do not limit the present invention.

### Reference Example 1

An acetate of NAcD2Nal-D4ClPhe-D3Pal-Ser-NMeTyr-DLys(Nic)-Leu-Lys(Nisp)-Pro-DAlaNH₂ (manufactured by TAP Pharmaceutical Products Inc., an LHRH antagonist compound) (480 mg) was added to a solution of a mixture of equal weight of glycolic acid (GA)-2-hydroxybutyric acid (HBA) copolymer (GA/HBA: 60/40 (mol%), Mw: 16,300) and polylactic acid (Mw: 4,200) (3.52 g) in dichloromethane (4.5 mL) and dissolved. The solution was cooled to 16°C and added to an aqueous solution of 0.1% polyvinyl alcohol (1,000 mL) which was previously adjusted to 16°C, and prepared an oil/water emulsion using a turbine-type homomixer at 7,000 rpm. The emulsion was stirred at room temperature for 3 hours to evaporate dichloromethane, and the oil phase was solidified and collected by a centrifuge (05PR-22, manufactured by Hitachi, Ltd.) at 2,000 rpm. This was dispersed again in distilled water and centrifuged to wash out the free drug. Collected microcapsules were dispersed again by adding small amount of distilled water, then D-mannitol (1 g) was added to the dispersion and the dispersion was lyophilized to give powder. The percentage of the LHRH antagonist compound encapsulated in the microcapsules was 88%.

About 15 mg of the microcapsules were dispersed in dispersion medium (distilled water in which 2.5 mg of carboxymethylcellulose, 0.5 mg of Polysolbate 80 and 25 mg of mannitol are dissolved) (0.5 mL) and the dispersion was administered subcutaneously to the dorsum of a 10 weeks-old male SD rat with a 22G injection needle (an administration dose as a LHRH antagonist: 3 mg/kg). After administration, blood was collected from the aorta inferior in each predetermined time and the rat was sacrificed, and the microcapsules remained at the site of administration were collected and the LHRH antagonist therein was quantified by HPLC, thereby confirmed that about 80% of the initial content of the LHRH antagonist compound was successively released for 4 weeks from the microcapsules. The concentration of testosterone in blood collected at each time was suppressed to not more than detection limit (0.05 ng/mL) for 4 weeks and recovered to about 50% of the mean value of the untreated normal rat (2.5 ng/mL) after 6 weeks, thereby the medicable period of the sustained release agent of the LHRH antagonist compound at this administration dose was determined to be 4 weeks.

### Example 1

Using 10 weeks-old male SD rat, the experiment was carried out for the following 4 groups. The second administration was carried out after 4 weeks of the first administration, and the testosterone in the serum of the collected blood before the second administration and after 1 day, 1 week, 2 weeks, 3 weeks, 4 weeks, 6 weeks, 8 weeks of the second administration was measured by RIA method.

| | Preparation of the first administration | Preparation of the second preparation |
|---|---|---|
| Group I | No administration | Reference Example |
| Group II | Leuplin injection 3.75 | Reference Example |
| Group III | No administration | Leuplin injection 3.75 |
| Group IV | Reference Example | Leuplin injection 3.75 |

The sustained release microcapsules of a LHRH antagonist for leuplin injection 3.75 (a one-month type sustained release microcapsules of an LHRH agonist, manufactured by Takeda Chemical Industries, Ltd., S130) obtained in Reference Example 1 was administrated subcutaneously by 3 mg/kg in terms of the respective compounds. The testosterone concentration below the detection limit of 0.05 ng/mL in the RIA, was regarded as 0.00 ng/mL. Table 1 shows the average concentration of testosterone and standard deviation (SD, n=5) (unit: ng/mL).

**Table 1**

| | Group I | Group II | Group III | Group IV |
|---|---|---|---|---|
| before the second administration | 2.48 (0.38) | 0.35 (0.12) | 2.49 (0.41) | 0.00 (0.00) |
| after one day of the second administration | 0.00 (0.00) | 0.00 (0.00) | 4.50 (0.60) | 0.07 (0.03) |
| after one week of the second administration | 0.00 (0.00) | 0.00 (0.00) | 0.30 (0.05) | 0.00 (0.00) |
| after two weeks of the second administration | 0.00 (0.00) | 0.00 (0.00) | 0.28 (0.05) | 0.00 (0.00) |
| after three weeks of the second administration | 0.00 (0.00) | 0.00 (0.00) | 0.29 (0.08) | 0.13 (0.07) |
| after four weeks of the second administration | 0.00 (0.00) | 0.00 (0.00) | 0.35 (0.12) | 0.22 (0.08) |
| after six weeks of the second administration | 1.26 (0.28) | 1.02 (0.41) | 0.52 (0.11) | 0.53 (0.13) |
| after eight weeks of the second administration | 2.40 (0.43) | 2.06 (0.42) | 1.34 (0.27) | 1.21 (0.14) |

The testosterone after 6 weeks or 8 weeks of the administration in the Groups I and II in which the sustained release microcapsules of a LHRH antagonist were administrated at the second administration, was more closer to the normal value of 2.5 ng/mL than that of the Groups III and IV in which the sustained release microcapsules of a LHRH agonist were administrated, and was more quickly recovered by administrating a preparation of the LHRH antagonist compound finally and the initiation of the drug cessation was more definitely determined. Then, the former administration mode is more suitable for an intermittent treatment.

### INDUSTRIAL APPLICABILITY

By using a preparation which contains a compound having a luteinizing hormone-releasing hormone antagonistic effect or a pharmacologically acceptable salt thereof or a preparation containing the compound (preferably a sustained release preparation), the concentration of a sex hormone (e.g., testosterone, LH, FSH, estrogen) after the medicable period of the compound having a luteinizing hormone-releasing hormone agonistic effect or an acetate thereof or a preparation containing the compound (preferably as compared to the groups of a single treatment with a sustained release preparation) is recovered more quickly, which makes it possible to definitely determine the drug cessation period in an intermittent treatment.

## Claims

1. Use of leuprorelin or an acetate thereof and a compound having a luteinizing hormone-releasing hormone antagonistic effect selected from NAcD2Nal-D4ClPhe-D3Pal-Ser-NMeTyr-DLys(Nic)-Leu-Lys(Nisp)-Pro-DAlaNH2, N(4H2-furoyl)Gly-D2Nal-D4ClPhe-D3Pal-Ser-NMeTyr-DLys(Nic)-Leu-Lys(Nisp)- Pro-DAlaNH₂, cetrorelix, ganirelix, antarelix, decirelix, azaline, antide, ramorelix and abarelix, or a pharmacologically acceptable salt thereof, for the preparation of a medicinal preparation for treating sex hormone-dependent diseases selected from prostate cancer, uterine cancer, breast cancer, glandula pituitaria tumor, bening prostatic hyperplasia, endometriosis, hysteromyoma, precocious puberty, dysmenorrhea, amenorrhea, premenstrual syndrome and multilocular ovarian syndrome, wherein leuprorelin or an acetate thereof is to be administered followed by the compound having a luteinizing hormone-releasing hormone antagonistic effect at the interval of not less than 1 day.

2. Use according to claim 1, wherein the compound having a luteinizing hormone-releasing hormone antagonistic effect is NAcD2Nal-D4ClPhe-D3Pal-Ser-NMeTyr-DLys(Nic)-Leu-Lys(Nisp)-Pro-DAlaNH₂ or a pharmacologically acceptable salt thereof.

3. Use according to claim 1, wherein the leuprorelin or an acetate thereof is to be used as an injectable preparation.

4. Use according to claim 1, wherein the leuprorelin or an acetate thereof is to be used as a sustained release preparation.

5. Use according to claim 1, wherein the leuprorelin or an acetate thereof is to be used as a preparation for nasal administration.

6. Use according to claim 1, wherein the compound having a luteinizing hormone-releasing hormone antagonistic effect is to be used as an injectable preparation.

7. Use according to claim 1, wherein the compound having a luteinizing hormone-releasing hormone antagonistic effect is to be used as a sustained release preparation.

8. Use according to claim 1, wherein the compound having a luteinizing hormone-releasing hormone antagonistic effect is to be used as a preparation for nasal administration.

9. Use according to claim 1, wherein the medicinal preparation is for maintaining a hormone therapy.

10. Use according to claim 1, wherein the medicinal preparation is for determining a drug cessation period.

11. Use according to claim 1, wherein the medicinal preparation is for recovering the concentration of a sex hormone in a living body.

12. Use according to claim 1, wherein the medicinal preparation is for maintaining sex hormone-dependency.

13. Use according to claim 1, wherein the sex hormone-dependent disease is prostate cancer.

## Patentansprüche

1. Verwendung von Leuprorelin oder einem Acetat desselben und einer Verbindung mit Luteinisierungs-Hormon-Freisetzungs-Hormon-antagonistischer Wirkung, ausgewählt aus NAcD2Nal-D4ClPhe-D3Pal-Ser-NMeTyr-DLys(Nic)-Leu-Lys(Nisp)-Pro-DAlaNH₂, N(4H2-Furoyl)Gly- D2Nal-D4ClPhe-D3Pal-Ser-NMeTyr-DLys(Nic)-Leu-Lys(Nisp)-Pro-DAlaNH₂, Cetrorelix, Ganirelix, Antarelix, Decirelix, Azalin, Antide, Ramorelix und Abarelix oder einem pharmakologisch verträglichen Salz desselben, zur Herstellung eines medizinischen Präparats zur Behandlung von sexualhormonabhängigen Erkrankungen, ausgewählt aus Prostatakrebs, Gebärmutterkrebs, Brustkrebs, Hypophysentumor, benigner Prostatahyperplasie, Endometriose, Uterusmyom, Pubertas praecox, Dysmenorrhoe, Amenorrhoe, prämenstruellem Syndrom und multilokulärem Ovarialsyndrom, wobei Leuprorelin oder ein Acetat desselben, gefolgt von der Verbindung mit Luteinisierungs-Hormon-Freisetzungs-Hormon-antagonistischer Wirkung, in Intervallen von nicht weniger als einem Tag zu verabreichen ist.

2. Verwendung gemäß Anspruch 1, wobei die Verbindung mit Luteinisierungs-Hormon-Freisetzungs-Hormon-antagonistischer Wirkung NAcD2Nal-D4ClPhe-D3Pal-Ser-NMeTyr-DLys(Nic)-Leu-Lys(Nisp)-Pro-DAlaNH₂ oder ein pharmakologisch verträgliches Salz desselben ist.

3. Verwendung gemäß Anspruch 1, wobei das Leuprorelin oder ein Acetat desselben als injizierbares Präparat zu verwenden ist.

4. Verwendung gemäß Anspruch 1, wobei das Leuprorelin oder ein Acetat desselben als Retardpräparat zu verwenden ist.

5. Verwendung gemäß Anspruch 1, wobei das Leuprorelin oder ein Acetat desselben als Präparat zur nasalen Verabreichung zu verwenden ist.

6. Verwendung gemäß Anspruch 1, wobei die Verbindung mit Luteinisierungs-Hormon-Freisetzungs-Hormon-antagonistischer Wirkung als injizierbares Präparat zu verwenden ist.

7. Verwendung gemäß Anspruch 1, wobei die Verbindung mit Luteinisierungs-Hormon-Freisetzungs-Hormon-antagonistischer Wirkung als Retardpräparat zu verwenden ist.

8. Verwendung gemäß Anspruch 1, wobei die Verbindung mit Luteinisierungs-Hormon-Freisetzungs-Hormon-antagonistischer Wirkung als Präparat zur nasalen Verabreichung zu verwenden ist.

9. Verwendung gemäß Anspruch 1, wobei das medizinische Präparat zur Einhaltung einer Hormontherapie bestimmt ist.

10. Verwendung gemäß Anspruch 1, wobei das medizinische Präparat zur Bestimmung einer Medikamentenabsetzungsperiode bestimmt ist.

11. Verwendung gemäß Anspruch 1, wobei das medizinische Präparat zur Wiederherstellung der Konzentration eines Sexualhormons in einem lebenden Körper bestimmt ist.

12. Verwendung gemäß Anspruch 1, wobei das medizinische Präparat zur Aufrechterhaltung der Sexualhormonabhängigkeit bestimmt ist.

13. Verwendung gemäß Anspruch 1, wobei die sexualhormonabhängige Erkrankung Prostatakrebs ist.

## Revendications

1. Utilisation de la leuproréline, ou de son acétate, et d'un composé qui a un effet antagoniste vis-à-vis de l'hormone de libération de la lutéinostimuline, choisi parmi les suivants :
NAcD2Nal-D4ClPhe-D3Pal-Ser-NMeTyr-DLys(Nic)-Leu-Lys(Nisp)-Pro-DAla-NH₂,
N(4H2-furoyl)Gly-D2Nal-D4ClPhe-D3Pal-Ser-NMeTyr-DLys(Nic)-Leu-Lys(Nisp)-Pro-DAla-NH₂,
cétrorélix, ganirelix, antarélix, décirélix, azaline, antide, ramorélix et abarélix,
ou d'un sel pharmacologiquement admissible d'un tel composé, en vue de préparer une préparation médicinale destinée au traitement de maladies dépendantes d'une hormone sexuelle, choisies parmi les suivantes :
cancer de la prostate, cancer de l'utérus, cancer du sein, tumeur de l'hypophyse, hyperplasie bénigne de la prostate, endométriose, hystéromyome, puberté précoce, dysménorrhée, aménorrhée,
syndrome prémenstruel, et syndrome des ovaires polykystiques, dans laquelle on administre la leuproréline ou son acétate, suivi(e), avec un intervalle d'au moins 1 jour, du composé qui a un effet antagoniste vis-à-vis de l'hormone de libération de la lutéinostimuline.

2. Utilisation conforme à la revendication 1, dans laquelle le composé qui a un effet antagoniste vis-à-vis de l'hormone de libération de la lutéinostimuline est le NAcD2Nal-D4ClPhe-D3Pal-Ser-NMeTyr-DLys(Nic)-Leu-Lys(Nisp)-Pro-DAla-NH₂, ou un sel pharmacologiquement admissible de ce composé.

3. Utilisation conforme à la revendication 1, dans laquelle la leuproréline ou son acétate doit être utilisé(e) sous forme de préparation injectable.

4. Utilisation conforme à la revendication 1, dans laquelle la leuproréline ou son acétate doit être utilisé(e) sous forme de préparation à libération prolongée.

5. Utilisation conforme à la revendication 1, dans laquelle la leuproréline ou son acétate doit être utilisé(e) sous forme de préparation pour administration par voie nasale.

6. Utilisation conforme à la revendication 1, dans laquelle le composé qui a un effet antagoniste vis-à-vis de l'hormone de libération de la lutéinostimuline doit être utilisé sous forme de préparation injectable.

7. Utilisation conforme à la revendication 1, dans laquelle le composé qui a un effet antagoniste vis-à-vis de l'hormone de libération de la lutéinostimuline doit être utilisé sous forme de préparation à libération prolongée.

8. Utilisation conforme à la revendication 1, dans laquelle le composé qui a un effet antagoniste vis-à-vis de l'hormone de libération de la lutéinostimuline doit être utilisé sous forme de préparation pour administration par voie nasale.

9. Utilisation conforme à la revendication 1, dans laquelle la préparation médicinale doit servir à l'entretien d'une hormonothérapie.

10. Utilisation conforme à la revendication 1, dans laquelle la préparation médicinale doit servir à la détermination d'une période d'arrêt de traitement par médicament.

11. Utilisation conforme à la revendication 1, dans laquelle la préparation médicinale doit servir au rétablissement de la concentration d'une hormone sexuelle chez un être vivant.

12. Utilisation conforme à la revendication 1, dans laquelle la préparation médicinale doit servir à l'entretien d'un état dépendant d'une hormone sexuelle.

13. Utilisation conforme à la revendication 1, dans laquelle la maladie dépendant d'une hormone sexuelle est un cancer de la prostate.
